Europäisches Patentamt

European Patent Office

Office européen des brevets

(19)

(11) Veröffentlichungsnummer: **0 170 020 B1**

# EUROPÄISCHE PATENTSCHRIFT

(12)

(45) Veröffentlichungstag der Patentschrift: **30.10.91**

(51) Int. Cl.⁵: **A61K 6/10, C08L 5/04**

(21) Anmeldenummer: **85107425.2**

(22) Anmeldetag: **15.06.85**

Die Akte enthält technische Angaben, die nach
dem Eingang der Anmeldung eingereicht wurden
und die nicht in dieser Patentschrift enthalten sind.

(54) **Abformmaterial und Verfahren zu dessen Herstellung.**

(30) Priorität: 30.06.84 DE 3424146

(43) Veröffentlichungstag der Anmeldung:
05.02.86 Patentblatt 86/06

(45) Bekanntmachung des Hinweises auf die
Patenterteilung:
30.10.91 Patentblatt 91/44

(84) Benannte Vertragsstaaten:
**AT BE CH DE FR GB IT LI NL SE**

(56) Entgegenhaltungen:
**EP-A- 0 048 123**
**WO-A-82/00650**
**FR-A- 2 553 999**
**GB-A- 1 344 377**

(73) Patentinhaber: **BAYER AG**

**W-5090 Leverkusen 1 Bayerwerk(DE)**

(72) Erfinder: **Kooke, Dieter**
**Kurlandweg 41**
**W-5653 Leichlingen(DE)**
Erfinder: **Schwabe, Peter, Dr.**
**Dudweiler Strasse 17**
**W-5090 Leverkusen 1(DE)**

EP 0 170 020 B1

**Beschreibung**

Die Erfindung betrifft ein verbessertes, staubfreies, pulverförmiges Abformmaterial auf Basis von Alginat, vorzugsweise für dentale Anwendungszwecke, sowie ein Verfahren zu dessen Herstellung durch Behandeln des pulverförmigen Materials mit bestimmten hydrophoben Flüssigkeiten.

Alginat-Abformmaterialien werden seit mehreren Jahrzehnten in der Zahnheilkunde z.B. zur Herstellung von Abformungen verwendet. Das pulverförmige Material wird dabei unmittelbar vor seiner Anwendung mit einer bestimmten Menge Wasser versetzt und zu einer Paste verrührt, die auf den Gegenstand aufgebracht wird, von dem ein Abdruck hergestellt werden soll. Nach einigen Minuten erstarrt die zunächst plastische Masse zu einem flexiblen formstabilen Gel, welches die Oberflächenkonturen des abzuformenden Objektes als Negativ wiedergibt und zur Herstellung eines Positivabdruckes, z.B. eines Gipsmodells des Objektes, dienen kann. Geeignete pulverförmige Alginatmassen werden z.B. in den US-Patenten 2 345 255, 2 390 137, 2 397 145, 2 422 497, 2 733 156, 2 878 129, 3 246 998 und Re 23 700, in GB-PS 518 596 sowie DE-OS 2 511 168 beschrieben.

Abformmassen gemäß diesem Stand der Technik haben den Nachteil, daß ihre Komponenten zur Staubbildung neigen und mit Wasser zum Teil schwer benetzbar sind. Beim Öffnen des Vorratsgefäßes bzw. beim Dosieren des Materials geht ein Teil des Alginats bzw. der anderen Bestandteile als sehr feinteiliger Staub verloren, was nicht nur zur Belästigung des Verarbeiters führt, sondern beim Einatmen des Staubes auch gesundheitsgefährdend sein kann. Darüber hinaus läßt sich das pulverförmige Material nur schlecht mit Wasser vermischen und schwimmt beim Einrühren längere Zeit auf der Oberfläche der Flüssigkeit.

Zur Vermeidung dieser Probleme wird in EP-A-00 58 203 vorgeschlagen, die pulverförmige Abdruckmasse oder zumindest eine Komponente hiervon mit einer Substanz zu beschichten, welche in Wasser löslich oder zumindest dispergierbar bzw. benetzbar ist. Gegenwärtig auf dem Markt befindliche Alginat-Abformmassen enthalten als derartiges Beschichtungsmittel hydrophile Polyether, z.B. Polypropylenglykol. Wie sich zeigte, lösen sich Abformmassen gemäß EP-A-00 58 203 nach der Aushärtung relativ schlecht vom abzuformenden Objekt; darüber hinaus weisen sie eine stumpfe Oberfläche auf und auch die Dispergierbarkeit in Wasser ist noch nicht voll befriedigend. Die Fr-A-2 553 999 schlägt vor, Abformmassen auf Alginatbasis hinsichtlich des Staubverhaltens durch die Zugabe von hydrophoben Flüssigkeiten aus der Gruppe der Kohlenwasserstoffe und Siliconöle ohne hydrophile Gruppe, mit einem Dampfdruck von nicht mehr als 420 Pa bei 20° C zu versetzen. Allderdings ist die Zugabe von Polyvinylpyrrolidon erforderlich, um die Verschlechterung der Benetzungseigenschaften der Abformmasse durch die Zugabe der hydrophoben Flüssigkeiten auszugleichen.

Es wurde nun gefunden, daß man verbesserte staubfreie Abformmassen mit größerer Zeichnungsschärfe und schneller Dispergierbarkeit in Wasser erhält, die geschmeidig und nach der Aushärtung leicht vom Zahn ablösbar sind, wenn man die pulverförmige Komponentenmischung mit Paraffin behandelt. Dies ist als besonders überraschend anzusehen, da nicht zu erwarten war, daß sich die an der Oberfläche mit dem sehr hydrophoben Paraffin beschichteten Partikel rasch in Wasser dispergieren lassen würden, ohne daß die Zugabe oberflächenaktiver Substanzen wie Polyvinylpyrrolidon erforderlich ist. Tatsächlich können mit anderen hydrophoben Substanzen wie z.B. Siliconölen (Polydimethylsiloxane) behandelte Alginatmassen wegen ihrer mangelnden Dispergierbarkeit in Wasser nicht zu Pasten verarbeitet werden.

Gegenstand der Erfindung ist somit ein pulverförmiges Abdruckmaterial, das beim Mischen mit Wasser eine zu fester Konsistenz aushärtende Paste ergibt, wobei das pulverförmige Material durch Zusatz eines Beschichtungsmittels staubfrei gemacht ist, dadurch gekennzeichnet, daß das Material als Zusatzstoff Paraffin enthält.

Die efindungsgemäßen Materialien enthalten 0,5 bis 7 Gew.-%, insbesondere 2,5 bis 5 Gew.-%, an Paraffin. Die Paraffine haben eine Viskosität von 10-100 000 mPas (cP)/20° C, insbesondere 20-10 000 mPas (cP)/20° C. Besonders bevorzugt werden erfindungsgemäß handelsübliche Paraffinöle eingesetzt.

Erfindungsgemäß werden vorzugsweise Abformmaterialien auf Alginatbasis staubfrei und besser dispergierbar gemacht, wie sie z.B. in den oben genannten Literaturstellen beschrieben werden. Im allgemeinen enthalten derartige Abformmaterialien ein lösliches Alginat (z.B. das Na- und/oder K-Salz von Alginsäure) in einer Menge von vorzugsweise 8-25 Gew.-%, insbesondere 10-17 Gew.-%, eine Metallverbindung, die mit Alginsäure ein wasserunlösliches Salz bildet (z.B. Pb-, Ca- oder Mg-Verbindungen wie MgO, MgCO$_3$ oder CaSO$_4$) in einer Menge von vorzugsweise 5 bis 40 Gew.-%, insbesondere 10-25 Gew.-%, einen Verzögerer für die Aushärtung (z.B. ein Alkaliphosphat, -diphosphat, -pyrophosphat oder -polyphosphat) in einer Menge von vorzugsweise 0,5-10 Gew.-%, insbesondere 1-5 Gew.-%, sowie einen Füllstoff (z.B. Gips, Kieselgur, Diatomeenerde, Ton, Talk, etc.) in einer Menge von vorzugsweise 25 bis 85 Gew.-%, insbesondere 40-75 Gew.-%. Gegebenenfalls können weitere Zuschlagstoffe wie z.B. Farb- und Geschmacksstoffe, sowie die

2

Verträglichkeit mit Gips verbessernde Verbindungen (z.B. K-fluortitanat oder K-fluorzirkonat) in der Abformmasse enthalten sein. Der Zusatz von oberflächenaktiven Substanzen als Hilfsstoffe zur Verbesserung der Benetzbarkeit der Abformmassen ist nicht erforderlich.

Die erfindungsgemäßen Abformmaterialien werden hergestellt, indem man dem pulverförmigen Gemisch der oben genannten Komponenten das Paraffin, vorzugsweise bei erhöhten Temperaturen des Mischgutes (z.B. 50-100° C, insbesondere 60-70° C) zusetzt. Dies kann auch in der Weise geschehen, daß nach Zusatz des Paraffins das Gemisch aufgeheizt wird. Weniger bevorzugt ist es, nur eine (oder einige) der Komponenten mit Paraffin zu behandeln und dann den restlichen Komponenten zuzugeben. Der Zusatz des Paraffins erfolgt zweckmäßigerweise in einem Schaufelmischer, z.B. einem Lödige-Mischer, dessen Wand mit Düsen versehen ist, durch welche das Paraffin auf das pulverförmige Gemisch aufgesprüht wird.

Die nachfolgenden Beispiele erläutern die Erfindung. Wenn nicht anders vermerkt, sind Mengenangaben als Gewichtsteile bzw. Gewichtsprozente zu verstehen.

Beispiele

Eine Alginat-Abformmasse wurde durch Vermischen folgender Komponenten hergestellt:

| | |
|---|---|
| Kaliumalginat | 15,0 % |
| $CaSO_4$ | 10,6 % |
| $MgCO_3$ | 1,5 % |
| $Na_4P_2O_7$ | 1,5 % |
| $K_2TiF_6$ | 3,4 % |
| Kaolin | 10,0 % |
| Talk | 2,1 % |
| Diatomeenerde | 55,9 % |

Das pulverförmige Material wurde in mehrere Portionen geteilt, die in folgender Weise mit verschiedenen Beschichtungsmitteln behandelt wurden: Das Gemisch wurde in einem Pulvermischer (Pflugscharmischer) vorgelegt, auf 60-70°C erwärmt und von oben mittels einer Sprühpistole mit dem jeweiligen Beschichungsmittel besprüht. Nach 10 Minuten wurde auf Kühlung umgestellt und abkühlen gelassen.

Die so beschichteten Proben wurden auf ihre relative Staubentwicklung geprüft. Das hierzu benutzte Gerät ist eine Kammer mit dem Abmessungen 50x50x50 cm, welche eine Einlaßdüse und eine Absaugvorrichtung mit einem Membranfilter aufweist. Die Pumpe an der Absaugvorrichtung wird eingeschaltet und auf einen Durchfluß von 27 l/min eingestellt. Über einen an der Einlaßdüse angebrachten Probentrichter werden 400 mg Probe durch Druckluftstoß in die Kammer eingeblasen und die Pumpe 4 Minuten später abgestellt. Das Gewicht des auf dem Membranfilter abgelagerten Staubes, multipliziert mit dem empirischen Faktor 9,4, ergibt die mittlere Staubkonzentration in der Kammer.

Die Ergebnisse (Staubkonzentration in mg/m³) sind in der nachstehenden Tabelle zusammengestellt (Mittelwerte aus 5 Messungen):

3

| Gew.-% Beschichtungsmittel | A | B | C | D | E |
|---|---|---|---|---|---|
| 1 | 408 | | | 263 | 376 |
| 2 | 395 | | | 235 | 328 |
| 3 | 386 | | 228 | 216 | 244 |
| 4 | 365 | | 222 | 197 | |
| 5 | 348 | 300 | 179 | 178 | |

A     Glycerin
B     Polydimethylsiloxan (500 mPas (cP)/20 °C)
C     Polydimethylsiloxan (100 mPas (cP)/20 °C)
D     Paraffinöl mit 180 mPas (cP)/23 °C (erfindungsgemäß)
E     Polyethylenglykol

Mit den Substanzen B bzw. C beschichtete Proben ließen sich nicht in Wasser einrühren.

Analoge Resultate wurden mit Paraffinöl der Viskosität 70 cP/23 °C bzw. den folgenden Alginat-Rezepturen (Angaben in Gew.-%) erhalten:

| | | | | | |
|---|---|---|---|---|---|
| K- bzw. Na-Alginat | 12,8 | 14,4 | 16,5 | 17,0 | 14,0 |
| $CaSO_4 \cdot 2H_2O$ | 2,8 | 7,8 | 28,0 | 9,4 | 11,0 |
| $MgO$ | 3,6 | 9,4 | 10,4 | 4,6 | |
| $NaF$ | | | | 0,7 | |
| $K_2TiF_6$ | 2,8 | | | 2,0 | 2,0 |
| $Na_4P_2O_7$ | 0,2 | 0,8 | 3,9 | | |
| $Na_3PO_4$ | | | | 0,7 | 2,0 |
| $Na_2SiF_6$ | | 3,1 | | | |
| $K_2ZrF_6$ | | | 3,8 | | |
| Al-Silikat | | | | | 9,0 |
| Kieselgur | | | | | 62,0 |
| Diatomeenerde | 77,6 | 64,5 | 37,4 | 65,5 | |

## Patentansprüche

Patentansprüche für folgende Vertragsstaaten: BE, CH, DE, FR, GB, IT, LI, NL, SE

1. Pulverförmiges Abformmaterial, bestehend aus einem Alginat und/oder einer Metallverbindung, die mit Alginsäure ein wasserunlösliches Salz bildet, einem Aushärtungsverzögerer, einem Füllstoff, gegebenenfalls üblichen Hilfsstoffen, dadurch gekennzeichnet, daß zumindest ein Teil des pulverförmigen Gemisches mit einem Paraffin mit einer Viskosität von 10 bis 100.000 mPas/20 °C beschichtet ist und der Paraffinanteil, bezogen auf die gesamte Mischung, 0,5 bis 7 Gew.-% beträgt.

2. Abformmaterial nach Anspruch 1, dadurch gekennzeichnet, daß es 2,5 bis 5 Gew-% Paraffin enthält.

3. Abformmaterial nach Ansprüchen 1 und 2, dadurch gekennzeichnet, daß das Paraffin eine Viskosität zwischen 20 und 10.000 mPas/20 °C aufweist.

4

4. Abformmaterial nach Ansprüchen 1 bis 3, dadurch gekennzeichnet, daß es als härtbare Komponente ein wasserlösliches Alginat enthält.

5. Verfahren zur Herstellung eines Abformmaterials nach Ansprüchen 1 bis 4, dadurch gekennzeichnet, daß man dem Gemisch der pulverförmigen Komponenten bzw. einem Teil hiervon unter Rühren Paraffin zusetzt.

**Patentansprüche für folgenden Vertragsstaat: AT**

1. Verfahren zur Herstellung eines pulverförmigen Abformmaterials, bestehend aus einem Alginat und/oder einer Metallverbindung, die mit Alginsäure ein wasserunlösliches Salz bildet, einem Aushärtungsverzögerer, einem Füllstoff, gegebenenfalls üblichen Hilfsstoffen, dadurch gekennzeichnet, daß zumindest ein Teil des pulverförmigen Gemisches mit einem Paraffin mit einer Viskosität von 10 bis 100.000 mPas/20°C beschichtet wird und der Paraffinanteil, bezogen auf die gesamte Mischung, 0,5 bis 7 Gew.-% beträgt und man dem Gemisch der pulverförmigen Komponenten bzw. einem Teil hiervon unter Rühren dieses Paraffin zusetzt.

2. Verfahren gemäß Anspruch 1, dadurch gekennzeichnet, daß der Paraffinanteil 2,5 bis 5 Gew.-% bezogen auf das gesamte Abformmaterial beträgt.

3. Verfahren gemäß Ansprüchen 1 und 2, dadurch gekennzeichnet, daß das Paraffin eine Viskosität zwischen 20 und 10.000 mPas/20°C aufweist.

4. Verfahren gemäß Ansprüchen 1 bis 3, dadurch gekennzeichnet, daß als härtbare Komponente ein wasserlösliches Alginat verwendet wird.

**Claims**
**Claims for the following Contracting States: BE, CH, DE, FR, GB, IT, LI, NL, SE**

1. Moulding material in the form of a powder, consisting of an alginate and/or of a metal compound which forms with alginic acid a salt which is insoluble in water, of a curing retardant, of a filler and, where appropriate, customary auxiliaries, characterised in that at least a portion of the mixture in the form of a powder is coated with a paraffin having a viscosity of 10 to 100,000 mPas/20°C, and the paraffin content, based on the total mixture, is 0.5 to 7% by weight.

2. Moulding material according to Claim 1, characterised in that it contains 2.5 to 5% by weight of paraffin.

3. Moulding material according to Claims 1 and 2, characterised in that the paraffin has a viscosity between 20 and 10,000 mPas/20°C.

4. Moulding material according to Claims 1 to 3, characterised in that it contains as the curable component an alginate which is soluble in water.

5. Process for the preparation of a moulding material according to Claims 1 to 4, characterised in that paraffin is added, with stirring, to the mixture of the components in the form of a powder, or to a portion thereof.

**Claims for the following Contracting State: AT**

1. Process for the preparation of a moulding material in the form of a powder, consisting of an alginate and/or of a metal compound which forms with alginic acid a salt which is insoluble in water, of a curing retardant, of a filler and, where appropriate, customary auxiliaries, characterised in that at least a portion of the mixture in the form of a powder is coated with a paraffin having a viscosity of 10 to 100,000 mPas/20°C, and the paraffin content, based on the total mixture, is 0.5 to 7% by weight, and this paraffin is added, with stirring, to the mixture of the components in the form of a powder, or to a portion thereof.

2. Process according to Claim 1, characterised in that the paraffin content is 2.5 to 5% by weight, based on the total moulding material.

3. Process according to Claims 1 and 2, characterised in that the paraffin has a viscosity between 20 and 10,000 mPas/20°C.

4. Process according to Claims 1 to 3, characterised in that an alginate which is soluble in water is used as the curable component.

**Revendications**
**Revendications pour les Etats contractants suivants: BE, CH, DE, FR, GB, IT, LI, NL, SE**

1. Matériau de moulage en poudre constitué d'un alginate et/ou d'un composé métallique qui forme avec l'acide alginique un sel insoluble dans l'eau, d'un retardateur de durcissement, d'une charge, le cas échéant de substances auxiliaires classiques, caractérisé en ce qu'au moins une partie du mélange en poudre est revêtue d'une paraffine ayant une viscosité de 10 à 100 000 mPa.s/20°C et la fraction de paraffine, par rapport au mélange total, s'élève à 0,5-7 % en poids.

2. Matériau de moulage suivant la revendication 1, caractérisé en ce qu'il contient 2,5 à 5 % en poids de paraffine.

3. Matériau de moulage suivant les revendications 1 et 2, caractérisé en ce que la paraffine présente une viscosité de 20 à 10 000 mPa.s/20°C.

4. Matériau de moulage suivant les revendications 1 à 3, caractérisé en ce qu'il contient un alginate hydrosoluble comme composant durcissable.

5. Procédé de production d'un matériau de moulage suivant les revendications 1 à 4, caractérisé en ce qu'on ajoute de la paraffine en agitant au mélange des composants en poudre ou à une partie de ce mélange.

**Revendications pour l'Etat contactant suivant: AT**

1. Matériau de moulage en poudre constitué d'un alginate et/ou d'un composé métallique qui forme avec l'acide alginique un sel insoluble dans l'eau, d'un retardateur de durcissement, d'une charge, le cas échéant de substances auxiliaires classiques, caractérisé en ce qu'au moins une partie du mélange en poudre est revêtue d'une paraffine ayant une viscosité de 10 à 100 000 mPa.s/20°C, et la fraction de paraffine, par rapport au mélange total, s'élève à 0,5-7 % en poids.

2. Matériau de moulage suivant la revendication 1, caractérisé en ce qu'il contient 2,5 à 5 % en poids de paraffine.

3. Matériau de moulage suivant les revendications 1 et 2, caractérisé en ce que la paraffine présente une viscosité de 20 à 10 000 mPa.s/20°C.

4. Matériau de moulage suivant les revendications 1 à 3, caractérisé en ce qu'il contient un alginate hydrosoluble comme composant durcissable.